# EUROPEAN PATENT APPLICATION

(11) **EP 1 970 119 A1**
(43) Date of publication of application: **17.09.2008**
(21) Application number: 05782599.4
(22) Date of filing: 24.08.2005
(51) Int. Cl.: B01J 31/12, B01J 31/14, B01J 31/02, B01J 29/00, C07D 317/36

(54) **CATALYTIC COMPOSITION FOR THE INSERTION OF CARBON DIOXIDE INTO ORGANIC COMPOUNDS**

(30) Priority: 25.08.2004 ES 200402112
(71) Applicant: Consejo Superior de Investigaciones Cientificas, 28006 Madrid (ES); UNIVERSIDAD POLITECNICA DE VALENCIA, 46022 Valencia (ES)
(72) Inventor: ÁLVARO RODRÍGUEZ, Mercedes Universidad Politécnica de Valencia, 46022 VALENCIA (ES); CARBONELL LLOPIS, Esther Universidad Politécnica de Valencia, 46022 VALENCIA (ES); CORMA CANOS, Avelino, Instituto de Tecnologia Quimica, Avda. Los Naranjos, s/n 46022 VALENCIA (ES); GÁRCIA GÓMEZ, Hermenegildo Universidad Politécnica de Valencia, 46022 VALENCIA (ES)
(74) Representative: Ungria Lopez, Javier
(86) International application number: PCT/ES2005/070124
(87) International publication number: WO 2006/032716

(57) **Abstract**

The invention relates to a catalytic composition comprising: a first component which is at least a component with one or more metals from groups 3A, 4A, 5A, 6A, 7A, 8, 1 B, 2B, 3B, 4B; and a second component selected from (1) at least one ionic liquid which consists of a compound formed by cations and anions and which is a liquid at ambient temperature, (ii) a matrix to which the first component is bound or on which it is supported, and (iii) a combination of the two. The invention relates to the use of said catalytic composition in a method for the insertion of carbon dioxide into an organic compound and, preferably, a compound selected from epoxides, acetals and orthoesters. The invention also relates to catalytic compositions comprising said metallic compounds.

## Description

### FIELD OF THE INVENTION

This invention comes within the field of preparation of new catalysts for the fixing of carbon dioxide.

### STATE OF THE PRIOR ART OF THE INVENTION

There currently exists great interest in developing industrial processes which use carbon dioxide as raw material. On the one hand, CO₂ is considered to be a renewable raw material which presents advantages over methane as a source of industrial compounds containing a single carbon atom. On the other hand, processes which consume atmospheric CO₂ can help to comply with the Kyoto agreements with regard to climate change, thereby helping to compensate for CO₂ emissions.

One reaction which has been described in the literature is the insertion of CO₂ into epoxides in order to give cyclic carbonates (equation 1). The precedents so far have used complexes of chromium and cobalt as "salen" ligands (N,N'-salicylaldehyde-ethylene-diimine) and phthalocyanines of chromium, in which none of the catalysts described are reusable.

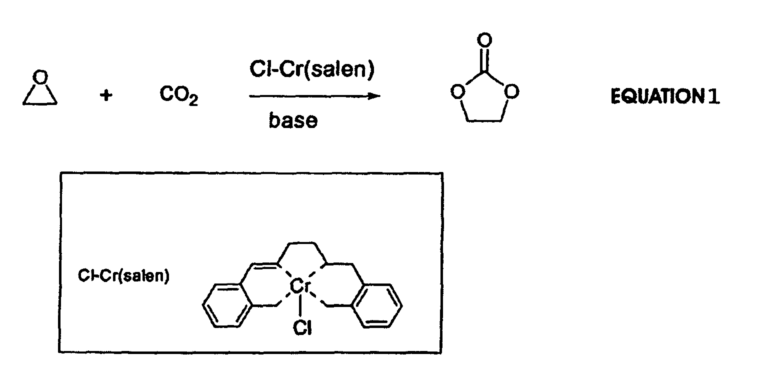

Moreover, searches that have been conducted have not revealed any precedents in the chemical literature on the reaction of acetals or orthoesters with carbon dioxide. In this case, the products that are formed are linear carbonates and the corresponding ketone (equation 2). The driving force which shifts the equilibrium, making the process thermodynamically possible, is the formation of a carbonyl group simultaneously with the reaction of the CO₂.

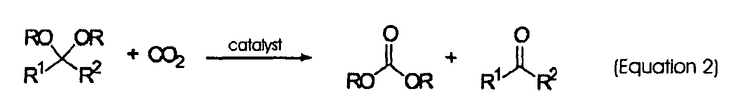

The carbonates can have applications as gasoline additives in order to improve the octane rating, as alternative industrial solvents to volatile organic solvents and as starting reagents in alkylation and carboxyalkylation reactions in substitution for halogenated compounds.

So, the present invention solves the problem of recovery of the catalysts which, owing to the dilution at which they are to be found in the reaction mixture, are very expensive to recover using known procedures, and on the other hand it succeeds in obtaining carbonates, which are useful in a great many applications, as has been stated earlier.

### DESCRIPTION OF THE INVENTION

The present invention refers first of all to the use of a catalytic composition which comprises:
- a first component which is at least one compound of one or more metals from groups 3A, 4A, 5A, 6A, 7A, 8, 1 B, 2B, 3B, 4B, and
- a second component selected from
- at least one ionic liquid which consists of a compound formed by cations and anions and which is a liquid at ambient temperature,
- a matrix to which the first component is bound or on which it is supported and
- a combination of the two,
in a method for the insertion of carbon dioxide into an organic compound.

In a preferred manner, said organic compound is selected from an epoxide, an acetal, an orthoester, and combinations of them.

According to the present invention, in the catalytic composition the first component is a compound of one of the aforementioned metals, which can be selected from a salt, a complex, a vinyl monomer of this metal complex, a polymer of said vinyl monomer, a copolymer of said vinyl monomer and combinations of them.

As metal salts with Lewis acid characteristics one can include, for example, aluminium trichloride, alkylaluminium compounds, zinc dichloride, iron chlorides, tin di- and tetrachlorides, titanium tetrachloride and titanium tetraalcoxides.

As a first component one can also use a compound, preferably a complex, of one of the aforementioned metals and preferably selected from chromium, cobalt, iron, vanadium and aluminium.

Said metal complex can have, for example, a Schiff base as ligand, which can be "salen" or it can be a Schiff base different from "salen".

According to the present invention, in the catalytic composition the first component is, in an especially preferred manner, a "salen" complex of a metal selected from chromium, cobalt, iron, vanadium and aluminium.

The metal complexes can be chiral when asymmetric carbons are introduced into the ligands. In these cases, the chiral complex can, for equation 1, insertion of CO₂ into epoxides, shown above, induce the formation of cyclic carbonates with enantiomeric excess.

A particular embodiment of this invention refers to the use of a catalytic composition which comprises:
- a first component which is at least one complex of one or more metals from groups 3A, 4A, 5A, 6A, 7A, 8, 1 B, 2B, 3B, 4B,
- a second component selected from
- at least one ionic liquid which consists of a compound formed by cations and anions and which is a liquid at ambient temperature,
- a matrix to which the first component is bound or on which it is supported and
- a combination of the two.
and a basic nucleophilic co-catalytic agent, in a method for the insertion of carbon dioxide into an organic compound, preferably selected from an epoxide, an acetal, an orthoester, and combinations of them.

Said basic nucleophilic co-catalyst can be a substituent which can be introduced into the metal complex or it can be a base. In the event that the nucleophilic co-catalyst is a substituent, it is preferably N-methylimidazol or N,N-dimethylaminopyridine, or a mixture of both.

According to additional particular embodiments of the catalytic composition, said basic nucleophilic agent is a base selected from tertiary amines, acetates of alkaline cations, acetates of alkaline earth cations, basic carbonates and basic solids.

The role of the said basic nucleophilic agent is to adapt the metal complex to the ionic liquid. In this regard, as well as complexes of Schiff type bases, these can also be modified for adapting them to the ionic liquid, improving their distribution coefficient and minimising losses of catalyst during the recovery of the products. This is done by introducing into the complex, as has been stated, a substituent, for example, imidazolium, as described in Scheme 2 (in which the symbol @ signifies a chemical bond between two components) for a specific embodiment, in the event that the first component is the "salen" aluminium complex.

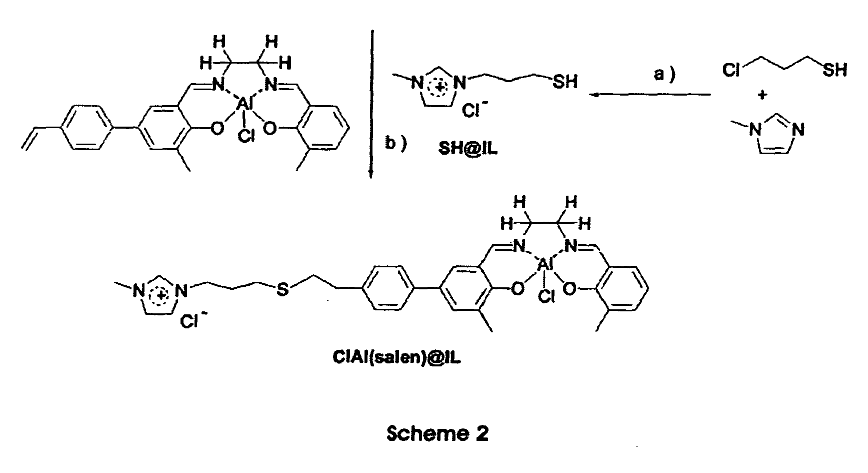

Depending on particular embodiments, one can therefore use as metal complexes, for example, complexes with Schiff bases of non-functionalised aluminium, dissolved in any ionic liquid, and complexes of Schiff bases of aluminium and chromium duly functionalised with imidazolium substituents.

According to the present invention, the first component of the composition acts as a Lewis acid, and can be recovered after the carbon dioxide insertion reaction and be reused, as well as permitting the design of a continual process for carrying out the transformations defined by equations 1 and 2 shown earlier.

The second component of the composition defined above can be selected from at least one ionic liquid, a matrix and a combination of both.

An ionic liquid is understood as being a compound which presents the liquid state at ambient temperature and which includes cations and anions. The products of the CO₂ insertion reaction are separated from the ionic liquid by any method of physical separation such as for example by extraction with a solvent immiscible with the ionic liquid such as hexane or ethers. Alternatively, the volatile products can be collected from the ionic liquid by evaporation.

According to preferred embodiments of the catalytic composition, said ionic liquid is a compound in which the cations are organic cations, and in an especially preferred manner said ionic liquid contains the imidazolium cation. The imidazolium cation can display one or more additional alkyl substituents, for example, one or more alkyl substituents selected from methyl, ethyl, propyl, butyl, hexyl, octyl, decyl, dodecyl, tetradecyl, hexadecyl and any of their fluoride derivatives.

In preferred embodiments of the catalytic composition, the cation is 1-methylimidazolium or 1-butyl-1-methylimidazolium.

Other embodiments of the composition have their ionic liquid in the form of a liquid which includes a cation selected from 1-ethyl-1-methylimidazolium, 1-butyl-3-ethyl-imidazolium and 1-hexyl-3-methylimidazolium.

For example, the imidazolium cation can be substituted or not in position 2 of the ring and with different counter anions, with hexafluorophosphate or tetrafluoroborate being two possible anions. Structure 1 corresponding to the hexafluorophosphate of 1-butyl-1-methylimidazolium represents a possible example.

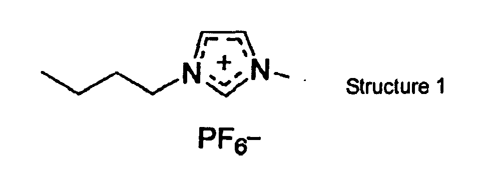

In general and for any embodiment of the invention, the ionic liquid can also have as counter anion acidic anions of aluminium or other Lewis acid which is prepared by the simple dissolution of two equivalents of the Lewis acid with the chloride of the ionic liquid under anhydrous conditions. An example is the anion Al₂Cl₇⁻, resulting from the reaction of two equivalents of anhydrous aluminium trichloride with the chloride of an organic cation of the imidazolium type (Equation 3).

2 AlCl₃ + Cl⁻ → Al₂Cl₇⁻ (Eq. 3)

When the ionic liquid includes the cation 1-butyl-1-methylimidazolium, the anion is preferably selected from tetrafluoroborate, phosphate, sulphate, trifluormethanosulphonate, bistrifluoromethanosulphoimidate, chloride, bromide, dialuminium heptachloride and combinations of them.

Other examples of ionic liquid can be one or more salts of quaternary ammonium salts, quaternary phosphonium salts and pyridinium salts.

A preferred embodiment of the invention is the use of a composition which comprises:
- a first component which is at least one compound of one or more metals from groups 3A, 4A, 5A, 6A, 7A, 8, 1 B, 2B, 3B, 4B, selected from a salt and a metal complex, and
- a second component which is at least one ionic liquid consisting of a compound formed from cations and anions and which is a liquid at ambient temperature, in a method of carbon dioxide insertion in an organic compound and preferably selected from an epoxide, an acetal, an orthoester, and combinations of them.

An especially preferred particular embodiment of the invention refers to the use of a catalytic composition which comprises:
- a first component which is the complex (salen)AI(III) chloride and N-methylimidazol and
- a second component which is at least one ionic liquid, in which the cation is 1-butyl-1-methylimidazolium.

In a preferred manner, the complex salen AI(IIII) chloride complex is enantiomerically pure.

According to additional particular embodiments of the invention, it refers to the use of a catalytic composition which comprises:
- a first component which is at least one compound of one or more metals from groups 3A, 4A, 5A, 6A, 7A, 8, 1 B, 2B, 3B, 4B, preferably a salt and a metal complex, and
- a second component which is a matrix selected from a polymeric organic solid and an inorganic solid.

Said matrix can be an inorganic solid selected, for example, from graphite, activated carbon and an allotropic form of carbon.

According to additional particular embodiments of the catalytic composition, the first component is a metal salt and said second component is a matrix selected from a polymeric organic solid and an inorganic solid.

According to additional particular embodiments of the catalytic composition, the first component is a metal complex and said second component is an inorganic solid to which said metal complex is covalently bound. Said first component is preferably a metal complex of "salen".

According to additional particular embodiments of the catalytic composition, the first component is a metal complex of "salen" and said second component is an organic polymer to which said metal complex of "salen" is covalently bound.

In addition to the covalent bond between the first component and the matrix, the first component - for example the metal salt - can be bound to the matrix electrostatically.

In any of the embodiments of the catalytic composition, the metal is preferably selected from aluminium (III), chromium (III), iron (III), vanadium (V) and Co (II).

The second component can also be silica, zeolites or other metal oxide, in such a way that, for example, said metal complex can be covalently anchored to it, or said complex can be bound to the matrix by means of electrostatic interactions. Said metal complex is preferably enantiomerically pure.

Additional particular embodiments of the invention refer to the use of a catalytic composition which comprises:
- a first component which is at least one compound of one or more metals from groups 3A, 4A, 5A, 6A, 7A, 8, 1 B, 2B, 3B, 4B, selected between a salt, a complex and combinations of both, and
- a second component which comprises
- at least one ionic liquid which consists of a compound formed by cations and anions and which is a liquid at ambient temperature, and
- a matrix to which the first component is bound or on which said first component is supported, in a method for the insertion of carbon dioxide into an organic compound, and preferably in a compound selected from epoxide, acetals and orthoesters.

In said embodiments, both the first component and the ionic liquid and the matrix can be any of the above described in the specification.

As an alternative to the ionic liquid, another organic solvent and in particular diethyl carbonate can be used as medium when a metal salt or metal complex is used supported on a polymeric or inorganic solid of large surface area.

Therefore, particular embodiments of the composition also refer to the use of a catalytic composition which comprises:
- a first component which is at least one compound of one or more metals from groups 3A, 4A, 5A, 6A, 7A, 8, 1 B, 2B, 3B, 4B, selected between a salt and a metal complex, and
- a second component which is a matrix to which the first component is bound, or on which it is supported and an organic solvent, such as for example an alkyl carbonate.

A method by which a "salen" complex can be anchored consists of modifying the ligand by means of introducing peripheral chloromethyl groups which act as reactive groups for carrying out the union with the solid previously functionalised as described in Schemes 3 and 4.

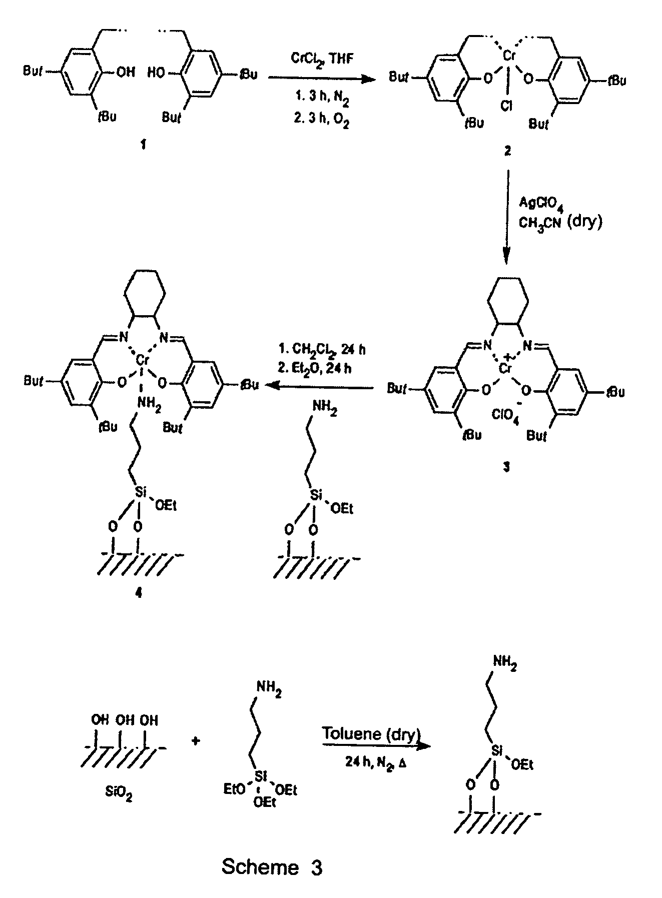

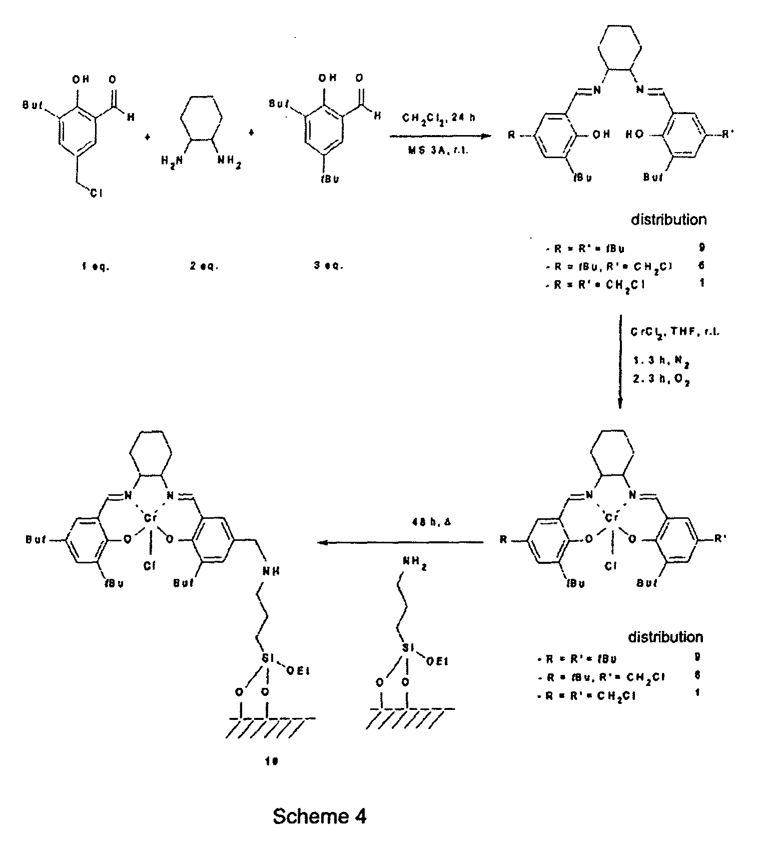

According to particular embodiments, when the second component is a matrix, as metal complexes one can use, for example, complexes with Schiff bases of non-functionalised aluminium, dissolved in any ionic liquid, and complexes of Schiff bases of aluminium and chromium duly functionalised in such a manner that they can be anchored to a support.

The catalytic composition of the invention can alternatively comprise as a second component one or more ionic liquids solely, or a matrix together with one or more ionic liquids.

When the second component is an ionic liquid, the composition works in the homogenous phase and said ionic liquid acts as a solvent, while when the second component contains a matrix, or a combination of at least one ionic liquid and a matrix, the composition operates in the heterogenous phase and the catalyst is a solid which remains insoluble during the reaction.

The homogenous reusable composition is based on the use of an ionic liquid and can furthermore contain a basic nucleophilic agent as co-catalyst.

Said insertion of CO₂ according to the present invention can be carried out by means of an operation selected from a continuous, semi-continuous and discontinuous reaction.

According to particular embodiments, the insertion of CO₂ takes place in a discontinuous reactor, with an interval of catalyst concentrations lying between 0.01 and 30 % by mols of catalyst with respect to the organic compound, in particular, epoxide, acetal or orthoester, which is the controlling reagent.

Said insertion of CO₂ consists, for example, of a reaction of a starting product selected from ethylene oxide, propylene oxide and styrene oxide which is converted into its corresponding carbonate.

Other examples of CO₂ insertion are a reaction of a starting product selected from methyl orthoformate, ethyl orthoformate, propyl orthoformate, isopropyl orthoformate and butyl orthoformate which is converted into the corresponding carbonate according to the equation: 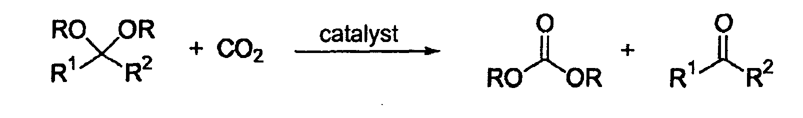 in which the radicals R, R¹ and R² can independently be hydrogen or an alkyl or aryl residue and preferably they are hydrogen or an alkyl residue of 1 to 20 carbon atoms.

The CO₂ insertion reaction is preferably carried out in a temperature range of between 20 °C and 180 °C.

The CO₂ insertion reaction is preferably carried out in a range of between atmospheric pressure and 150 bar.

The concentration of catalyst when referring to a reaction system in a discontinuous reactor lies between 0.01 and 30 % by mols with respect to the controlling reagent.

The present invention furthermore refers to a catalytic composition which comprises:
- a first component which is at least one compound of one or more metals from groups 3A, 4A, 5A, 6A, 7A, 8, 1 B, 2B, 3B, 4B, and
- a second component selected from
   - at least one ionic liquid which consists of a compound formed by cations and anions and which is a liquid at ambient temperature, and
   - a matrix to which the first component is bound or on which it is supported, together with at least one ionic liquid as defined earlier.

Both the first component, in other words, the metal compound, and the matrix, and the ionic liquid or liquids can be the same as have been defined earlier in this specification.

According to a particular embodiment of the invention, in the catalytic composition said first component is a compound selected from a salt, a complex, a vinyl monomer of this metal complex, a polymer of said vinyl monomer, a copolymer of said vinyl monomer and combinations of them. Said first component is preferably a compound of a metal selected from chromium, cobalt, iron, vanadium and aluminium, and more preferably yet is a complex of one of these metals.

According to additional particular embodiments of the composition, the first component is a metal complex which said has a Schiff base as ligand, which can be a "salen" ligand or it can be a Schiff base different from "salen".

Additional particular embodiments refer to compositions in which the first component is a complex with a "salen" ligand of a metal selected from chromium, cobalt, iron, vanadium and aluminium.

An additional particular object is a catalytic composition which comprises:
- a first component which is at least one complex of one or more metals from groups 3A, 4A, 5A, 6A, 7A, 8, 1 B, 2B, 3B, 4B,
- a second component which comprises a matrix to which the first component is bound or on which it is supported, together with at least one ionic liquid as defined earlier,
and a basic nucleophilic co-catalytic agent.

Said basic nucleophilic co-catalyst is preferably a ligand selected from N-methylimidazol or N,N-dimethylaminopyridine, or a mixture of both, or a base selected from tertiary amines, acetates of alkaline cations, acetates of alkaline earth cations, basic carbonates and basic solids.

In the catalytic composition of the invention, said ionic liquid is preferably a compound in which the cations are organic cations. A preferred cation is a cation selected from the imidazolium cation and the imidazolium cation with one or more additional alkyl substituents. Said alkyl substituents are preferably selected from methyl, ethyl, propyl, butyl, hexyl, octyl, decyl, dodecyl, tetradecyl, hexadecyl and any of their fluoride derivatives.

Examples of cations are 1-methylimidazolium, 1-butyl-1-methylimidazolium, 1-ethyl-1-methylimidazolium, 1-butyl-3-ethyl-imidazolium and 1-hexyl-3-methylimidazolium.

Examples of ionic liquid are a liquid that includes the cation 1-butyl-1-methylimidazolium, and an anion selected from tetrafluoroborate, phosphate, sulphate, trifluormethanosulphonate, bistrifluoromethanosulpho-imidate, chloride, bromide, dialuminium heptachloride and combinations of them.

The ionic liquid can also be one or more salts of quaternary ammonium salts or quaternary phosphonium and pyridinium salts.

An additional preferred embodiment refers to a catalytic composition which comprises:
- a first component which is at least one compound of one or more metals from groups 3A, 4A, 5A, 6A, 7A, 8, 1 B, 2B, 3B, 4B, selected from a salt and a metal complex, and
- a second component which is at least one ionic liquid consisting of a compound formed from cations and anions and which is a liquid at ambient temperature.

An especially preferred composition is one which comprises:
- a first component which is the complex (salen)AI(III) chloride and N-methylimidazol and
- a second component which is at least one ionic liquid, in which the cation is 1-butyl-1-methylimidazolium.

In an especially preferred manner, the complex salen AI(IIII) chloride complex is enantiomerically pure.

An additional preferred particular embodiment refers to a catalytic composition which comprises:
- a first component which is at least one compound of one or more metals from groups 3A, 4A, 5A, 6A, 7A, 8, 1 B, 2B, 3B, 4B, selected between a salt, a complex, and combinations of both, and
- a second component which is a matrix selected between a polymeric organic solid and an inorganic solid, together with at least one ionic liquid as defined earlier.

In any embodiment of the composition, said matrix can be an inorganic solid selected, for example, from graphite, activated carbon and an allotropic form of carbon.

In particular embodiments of the composition, the first component is a metal salt and said second component is a matrix selected from a polymeric organic solid and an inorganic solid.

According to other particular embodiments, the first component is a metal complex and said second component is an inorganic solid to which said metal complex is covalently bound.

The catalytic composition of the invention preferably has as the first component a metal complex of "salen".

Other examples of catalytic composition have as the first component a metal complex of "salen" and as the second component an organic polymer to which said metal complex of "salen" is covalently bound.

The catalytic composition can, according to additional embodiments, include as the metal complex a metal salt which is bound to the matrix electrostatically.

Other examples of catalytic composition have as their first component a metal complex which is covalently anchored to a matrix selected from silica, zeolites or other metal oxide.

The metal complex or the metal salt can be covalently anchored to the silica, zeolites or other metal oxide by means of electrostatic interactions.

In a preferred manner in the catalytic composition, the metal compound is an enantiomerically pure metal complex.

Finally, the present invention also refers to a method of insertion of carbon dioxide in an organic compound, in which a catalytic composition which comprises:
- a first component which is at least one compound of one or more metals from groups 3A, 4A, 5A, 6A, 7A, 8, 1 B, 2B, 3B, 4B, and
- a second component selected between
   - at least one ionic liquid which consists of a compound formed by cations and anions and which is a liquid at ambient temperature,
   - a matrix to which the first component is bound or on which it is supported and
   - and combination of both;.
is placed in contact with said organic compound, preferably an epoxide, an acetal or an orthoester, in order to carry out an insertion reaction in that organic compound.

In said method, both the first component of the composition and the second component can be those described above in this specification.

### EXAMPLES

Described below are some examples of embodiment of the invention:

### Example 1:

In a preferred embodiment, (salen)AI(III) chloride (30 mg) is used as catalyst and N-methylimidazol (10 µl) as co-catalyst, dissolved in 1-butyl-3-methylimidazol hexafluorophosphate (0.5 ml) and as reagent ethylene oxide (3.56 mmol), the system being charged in an autoclave which operates with CO₂ and working at a temperature of 80 °C and 100 bars. Under these conditions and after proceeding to recover the reaction products with hexane a conversion of 60% and a selectivity to carbonate of 60% is obtained. The system can be reused after evacuating the ionic liquid in order to eliminate residues of hexane.

Alternatively, the product can be recovered by heating the ionic liquid at 120 °C and condensing the vapours.

### Example 2:

The diethyl acetal of formaldehyde (0.5 ml) together with tributylamine and (salen)AI(III) chloride duly anchored to silica following the sequence of Scheme 4 for the case of chromium (30 mg) are introduced into an autoclave of volume 50 ml which is charged with CO₂ in such way that at 80 °C the system has a pressure of 100 bars. After 6 h of reaction, the autoclave is left to cool and is discharged, with diethyl carbonate being obtained as the reaction product with a yield of 30%.

### Example 3:

In the third example, a chiral complex of aluminium (III) salen, covalently anchored to a polyester matrix [AI(salen) / PS], was prepared, using as support 0.6 g of a co-polymer of styrene and divinylbenzene (2% by weight of 1,4-divinylbenzene) which is partially substituted with aminomethyl groups (1.6 mmol of N x g⁻¹). This polymer was added to a solution of 2,6-diformyl-4-tert-butylphenol (216 mg, 1.05 mmol) in ethanol (10 mL).

The polymer and dialdehyde mixture was magnetically stirred under reflux for 2 h. The solid was eliminated by filtration and extracted with dichloromethane in a Soxhlet equipment for 24 h, and was finally dried under a vacuum. In a second stage, the solid, functionalised with aldehyde groups, was made to react with (R,R)-1,2-diaminocyclohexane (120 mg, 1.05 mmol) in ethanol (10 mL), with the suspension being stirred for 2 h. This solid is filtered and extracted in Sohxlet with dichloromethane and dried under a vacuum. The solid obtained in the previous stage containing amine groups is made to react with a solution of 3,5-tert-butylsalicylaldehyde (246 mg, 1.05 mmol) in ethanol (10 mL), with the mixture being stirred under reflux for 2 h. After that time, the solid was recovered by filtration, washed and dried.

The addition of aluminium in order to obtain the chiral catalyst AI(salen) /PS is carried out at ambient temperature adding dichloromethane (10 mL) and diethylaluminium chloride (1 mL, 1.8. mmol, 1.8 M solution in toluene) in a dry flask under an atmosphere of dry nitrogen. After 4 h, the solid is gathered, extracted and dried. The content of aluminium was 1.26 mmol AI x g⁻¹ which was determined by atomic absorption spectroscopy.

With this catalyst AI(salen) /PS, which is one of the possible embodiments of this invention, the CO₂ insertion reaction into styrene oxide (4 ml) was carried out. This reaction was charged in an autoclave (125 ml) together with the catalyst AI(salen) /PS (0.40 % mol) and N-methylimidazol (3.6 % mol) as co-catalyst at a pressure of 100 bars and was mechanically stirred at 80 °C and 300 rpm. The corresponding cyclic carbonate was obtained with a yield of 68%, a selectivity of greater than 95% and an enantiomeric excess of greater than 40%.

The following table shows some results obtained with examples of catalytic compositions according to the invention.

| experiment | catalyst [substrate-to-catalyst mol %] | Mass balance (%) | Conversion^{a} (%) | Yield (%) | TON^{g} |
|---|---|---|---|---|---|
| 1 | AI (salen ) [0.1] | 91 | 19 | 17 | 9 |
| 2 | AI (salen )^{b} [0.1] | 100 | 20 | 17 | 9 |
| 3 | AI (salen )^{c} [0.1] | 98 | 4 | 3 | 10 |
| 4 | AI (salen )^{d} [0.1] | 97 | 4 | 3 | 10 |
| 5 | AI (salen) / PEA (use 1) [0.14] | 87 | 22 | 19 | 47 |
| 6 | AI (salen) / PEA (use 2) [0.14] | 95 | 9 | 6 | 5 |
| 7 | AI (salen) / PEA (use 3) [0.14] | 92 | 9 | 6 | 5 |
| 8 | AI (salen) / PEA^{e} [0.14] | 98 | 8 | 8 | 0 |
| 9 | AI (salen) / PS (use 1) [0.44] | 100 | 9 | 9 | |
| 10 | AI (salen) / PS (use 3) [0.44] | 97 | 9 | 9 | |
| 11 | [PEG-AI-CsCO₃]^{f} [0.1] | 86^{f} | 15 | 11 | 8 |
| ^{a} Based on the disappearance of styrene. | | | | | |
| ^{b} dimethylaminopyridine was used in place of N-methylimidazol. | | | | | |
| ^{c} P = 20 bar. | | | | | |
| ^{d} 2 eq of N-methylimidazol. | | | | | |
| ^{e} dimethylaminopyridine polymer was used as co-catalyst. | | | | | |
| ^{f} 1 % of polymer was observed as by-product. | | | | | |
| ^{g} number of catalytic cycles per metal atom. | | | | | |

## Claims

1. Use of a catalytic composition which comprises:
- a first component which is at least one compound of one or more metals from groups 3A, 4A, 5A, 6A, 7A, 8, 1 B, 2B, 3B, 4B, and
- a second component selected from
- at least one ionic liquid which consists of a compound formed by cations and anions and which is a liquid at ambient temperature,
- a matrix to which the first component is bound or on which it is supported, and
- a combination of the two,
in a method for the insertion of carbon dioxide into an organic compound.

2. Use according to claim 1, **characterised in that** said organic compound is selected from epoxides, acetals and orthoesters, and combinations of them.

3. Use according to claim 1 or 2, **characterised in that** said first component is a compound selected from a salt, a complex, a vinyl monomer of this metal complex, a polymer of said vinyl monomer, a copolymer of said vinyl monomer and combinations of them.

4. Use according to claim 1 or 3, **characterised in that** the first component is a compound of a metal selected from chromium, cobalt, iron, vanadium and aluminium.

5. Use according to claim 1 or 4, **characterised in that** the first component is a metal complex which has a Schiff base as ligand.

6. Use according to claim 1 or 4, **characterised in that** the first component is a complex of a metal selected from chromium, cobalt, iron, vanadium and aluminium and which has "salen" as ligand.

7. Use according to claim 1 or 3, **characterised in that** said catalytic composition comprises:
- a first component which is at least one complex of one or more metals from groups 3A, 4A, 5A, 6A, 7A, 8, 1 B, 2B, 3B, 4B,
- a second component selected from
- at least one ionic liquid which consists of a compound formed by cations and anions and which is a liquid at ambient temperature,
- a matrix to which the first component is bound or on which it is supported, and
- a combination of the two,
and a basic nucleophilic co-catalytic agent.

8. Use according to claim 7, **characterised in that** the basic nucleophilic co-catalyst is selected between
- a ligand selected between N-methylimidazol, N,N-dimethylaminopyridine and a mixture of both, and
- a base selected from tertiary amines, acetates of alkaline cations, acetates of alkaline earth cations, basic carbonates and basic solids.

9. Use according to claim 1 or 7, **characterised in that** said ionic liquid is a compound in which the cations are organic cations.

10. Use according to claim 1 or 9, **characterised in that** said ionic liquid contains a cation selected between an imidazolium cation and an imidazolium cation which displays one or more additional alkyl substituents.

11. Use according to one of claims 9 or 10, **characterised in that** the imidazolium cation displays one or more alkyl substituents selected from methyl, ethyl, propyl, butyl, hexyl, octyl, decyl, dodecyl, tetradecyl, hexadecyl and any of their fluoride derivatives.

12. Use according to one of claims 9 to 11, **characterised in that** the cation is selected between 1-methylimidazolium, 1-butyl-1-methylimidazolium, 1-ethyl-1-methylimidazolium, 1-butyl-3-ethyl-imidazolium and 1-hexyl-3-methylimid-azolium.

13. Use according to one of claims 9 to 12, **characterised in that** the ionic liquid comprises the cation 1-butyl-1-methylimidazolium and an anion selected among tetrafluoroborate, phosphate, sulphate, trifluormethanosulphonate, bistrifluoromethanosulphoimidate, chloride, bromide, dialuminium heptachloride and combinations of them.

14. Use according to claim 1 or 9, **characterised in that** said ionic liquid is selected from one or more salts of quaternary ammonium salts, quaternary phosphonium salts and pyridinium salts.

15. Use according to claim 1 or 12, **characterised in that** said composition comprises:
- a first component which is at least one compound of one or more metals from groups 3A, 4A, 5A, 6A, 7A, 8, 1 B, 2B, 3B, 4B, selected from a salt and a metal complex, and
- a second component which is at least one ionic liquid consisting of a compound formed from cations and anions and which is a liquid at ambient temperature.

16. Use according to claim 1 or 15, **characterised in that** said composition comprises:
- a first component which is the complex (salen)AI(III) chloride and N-methylimidazol and
- a second component which is an ionic liquid, in which the cation is 1-butyl-1-methylimidazolium.

17. Use according to claim 1, **characterised in that** said composition comprises:
- a first component which is at least one compound of one or more metals from groups 3A, 4A, 5A, 6A, 7A, 8, 1 B, 2B, 3B, 4B, selected between a salt, a complex and combinations of both, and
- a second component which is a matrix selected from a polymeric organic solid and an inorganic solid.

18. Use according to claim 1 or 17, **characterised in that** said matrix is an inorganic solid selected among graphite, activated carbon and an allotropic form of carbon.

19. Use according to claim 1 or 17, **characterised in that** said first component is a metal salt and said second component is a matrix selected from a polymeric organic solid and an inorganic solid.

20. Use according to claim 1 or 17, **characterised in that** said first component is a metal complex and said second component is an inorganic solid to which said metal complex is covalently bound.

21. Use according to claim 1 or 17, **characterised in that** said first component is a metal complex which has a "salen" ligand and said second component is an organic polymer to which said metal complex of "salen" is covalently bound.

22. Use according to claim 1 or 17, **characterised in that** the metal compound is a metal salt and is bound to the matrix electrostatically.

23. Use according to claim 3 or 17, **characterised in that** said metal complex is covalently anchored to a matrix selected between silica, zeolites and other metal oxide.

24. Use according to claim 3 or 22, **characterised in that** the metal complex or the metal salt is bound to silica, zeolites or other metal oxide by means of electrostatic interactions.

25. Use according to claim 1, **characterised in that** said composition comprises:
- a first component which is at least one compound of one or more metals from groups 3A, 4A, 5A, 6A, 7A, 8, 1 B, 2B, 3B, 4B, selected between a salt, a complex and combinations of both, and
- a second component which comprises
- at least one ionic liquid which consists of a compound formed by cations and anions and which is a liquid at ambient temperature, and
- a matrix to which the first component is bound or on which said first component is supported.

26. Use of a catalytic composition according to claim 1, **characterised in that** said composition comprises:
- a first component which is at least one compound of one or more metals from groups 3A, 4A, 5A, 6A, 7A, 8, 1 B, 2B, 3B, 4B, selected between a salt and a metal complex, and
- a second component which a matrix to which the first component is bound, or on which it is supported and an organic solvent.

27. Use of a catalytic composition according to one of claims 1 to 26, **characterised in that** the insertion of CO₂ is produced by means of an operation selected from a continuous, semi-continuous and discontinuous reaction.

28. Use of a catalytic composition according to one of claims 1 to 26, **characterised in that** the insertion of CO₂ takes place in a discontinuous reactor, with an interval of catalyst concentrations lying between 0.01 and 30 % by mols of catalyst with respect to said organic compound.

29. Use of a catalytic composition according to one of claims 1 to 28, **characterised in that** said insertion of CO₂ is a reaction of a starting product selected from ethylene oxide, propylene oxide and styrene oxide which is converted into its corresponding carbonate.

30. Use of a catalytic composition according to one of claims 1 to 28, **characterised in that** said CO₂ insertion is a reaction of a starting product selected from methyl orthoformate, ethyl orthoformate, propyl orthoformate, isopropyl orthoformate and butyl orthoformate which is converted into the corresponding carbonate according to the equation: 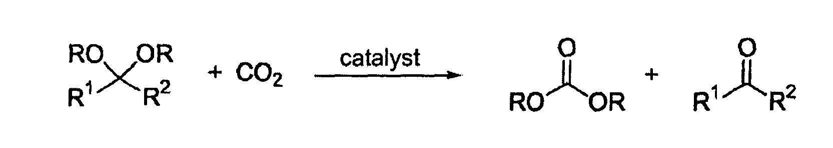 in which the radicals R, R¹ and R² are independently selected between hydrogen, an alkyl residue of 1 to 20 carbon atoms and an aryl residue.

31. Use of a catalytic composition according to one of claims 1 to 30, **characterised in that** said CO₂ insertion is carried out in a temperature range of between 20 °C and 180 °C.

32. Use of a catalytic composition according to one of claims 1 to 30, **characterised in that** said CO₂ insertion reaction is carried out in a range of between atmospheric pressure and 150 bar.

33. A catalytic composition **characterised in that** it comprises:
- a first component which is at least one compound of one or more metals from groups 3A, 4A, 5A, 6A, 7A, 8, 1 B, 2B, 3B, 4B, and
- a second component selected from
- at least one ionic liquid which consists of a compound formed by cations and anions and which is a liquid at ambient temperature, and
- a matrix to which the first component is bound or on which it is supported, together with at least one ionic liquid as defined earlier.

34. A catalytic composition according to claim 33, **characterised in that** said first component is a compound selected from a salt, a complex, a vinyl monomer of this metal complex, a polymer of said vinyl monomer, a copolymer of said vinyl monomer and combinations of them.

35. A catalytic composition according to claim 33, **characterised in that** the first component is a compound of a metal selected from chromium, cobalt, iron, vanadium and aluminium.

36. A catalytic composition according to claim 33, **characterised in that** the first component is a metal complex which has a Schiff base as ligand.

37. A catalytic composition according to claim 33, **characterised in that** the first component is a complex with a "salen" ligand of a metal selected from chromium, cobalt, iron, vanadium and aluminium.

38. A catalytic composition according to claim 33, **characterised in that** it comprises:
- a first component which is at least one complex of one or more metals from groups 3A, 4A, 5A, 6A, 7A, 8, 1 B, 2B, 3B, 4B,
- a second component which comprises a matrix to which the first component is bound or on which it is supported, together with at least one ionic liquid as defined earlier,
and a basic nucleophilic co-catalytic agent.

39. A catalytic composition according to claim 38, **characterised in that** the basic nucleophilic co-catalyst is selected between
- a ligand selected from N-methylimidazol, N,N-dimethylaminopyridine and a mixture of both, and
- a base selected from tertiary amines, acetates of alkaline cations, acetates of alkaline earth cations, basic carbonates and basic solids.

40. A catalytic composition according to claim 33 or 38, **characterised in that** said ionic liquid is a compound in which the cations are organic cations.

41. A catalytic composition according to claim 33 or 38, **characterised in that** said ionic liquid comprises a cation selected between the imidazolium cation and the imidazolium cation with one or more additional alkyl substituents.

42. A catalytic composition according to one of claims 40 or 41, **characterised in that** the imidazolium cation displays one or more alkyl substituents selected from methyl, ethyl, propyl, butyl, hexyl, octyl, decyl, dodecyl, tetradecyl, hexadecyl and any of their fluoride derivatives.

43. A catalytic composition according to one of claims 41 or 42, **characterised in that** the cation is selected from 1-methylimidazolium, 1-butyl-1-methylimidazolium, 1-ethyl-1-methylimidazolium, 1-butyl-3-ethylimidazolium and 1-hexyl-3-methylimidazolium.

44. A catalytic composition according to claim 41 or 43, **characterised in that** the ionic liquid comprises the cation 1-butyl-1-methylimidazolium, and an anion selected from tetrafluoroborate, phosphate, sulphate, trifluormethanosulphonate, bistrifluoromethanosulpho-imidate, chloride, bromide, dialuminium heptachloride and combinations of them.

45. A catalytic composition according to claim 33 or 38, **characterised in that** said ionic liquid is selected from one or more salts of quaternary ammonium salts, quaternary phosphonium salts and pyridinium salts.

46. A catalytic composition according to claim 33, **characterised in that** it comprises:
- a first component which is at least one compound of one or more metals from groups 3A, 4A, 5A, 6A, 7A, 8, 1 B, 2B, 3B, 4B, selected from a salt and a metal complex, and
- a second component which is at least one ionic liquid consisting of a compound formed from cations and anions and which is a liquid at ambient temperature.

47. A catalytic composition according to claim 33 or 46, **characterised in that** it comprises:
- a first component which is the complex (salen)AI(III) chloride and N-methylimidazol and
- a second component which is an ionic liquid, in which the cation is 1-butyl-1-methylimidazolium.

48. A catalytic composition according to claim 47, **characterised in that** the complex salen AI(IIII) chloride complex is enantiomerically pure.

49. A catalytic composition according to claim 33, **characterised in that** it comprises:
- a first component which is at least one compound of one or more metals from groups 3A, 4A, 5A, 6A, 7A, 8, 1 B, 2B, 3B, 4B, selected between a salt, a complex, and combinations of both, and
- a second component which is a matrix selected between a polymeric organic solid and an inorganic solid, together with at least one ionic liquid as defined above.

50. A catalytic composition according to claim 33 or 49, **characterised in that** said matrix is an inorganic solid selected from graphite, activated carbon and an allotropic form of carbon.

51. A catalytic composition according to claim 33 or 49, **characterised in that** said first component is a metal salt and said second component is a matrix selected from a polymeric organic solid and an inorganic solid.

52. A catalytic composition according to claim 33 or 49, **characterised in that** said first component is a metal complex and said second component is an inorganic solid to which said metal complex is covalently bound.

53. A catalytic composition according to claim 33 or 49, **characterised in that** said first component is a metal complex of "salen".

54. A catalytic composition according to claim 33 or 49, **characterised in that** said first component is a metal complex of "salen" and the second component is an organic polymer to which said metal complex of "salen" is covalently bound.

55. A catalytic composition according to claim 33 or 49, **characterised in that** the metal compound is a metal salt and is bound to the matrix electrostatically.

56. A catalytic composition according to claim 33 or 49, **characterised in that** said metal complex is covalently anchored to a matrix selected from silica, zeolites or other metal oxide.

57. A catalytic composition according to claim 33 or 49, **characterised in that** the metal complex or the metal salt is covalently anchored to the silica, zeolites or other metal oxide by means of electrostatic interactions.

58. A catalytic composition according to claim 33 or 49, **characterised in that** said metal compound is an enantiomerically pure metal complex.
